# EUROPEAN PATENT SPECIFICATION

(11) **EP 3 607 900 B1**
(45) Date of publication and mention of the grant of the patent: **09.06.2021**
(21) Application number: 19190525.6
(22) Date of filing: 07.08.2019
(51) Int. Cl.: A61B 17/32

(54) **ULTRASONIC SURGICAL INTEGRATED KNIFE**
CHIRURGISCHE INTEGRIERTE ULTRASCHALLKLINGE
COUTEAU CHIRURGICAL INTÉGRÉ À ULTRASONS

(30) Priority: 08.08.2018 CN 201810897869
(43) Date of publication of application: 12.02.2020
(73) Proprietor: BEIJING ANHEJIALIER TECHNOLOGY CO., LTD., Beijing (CN)
(72) Inventor: GAO, Zanjun, Beijing, Beijing 100176 (CN); XUE, Wanchao, Beijing, Beijing 100176 (CN); ZOU, Jianlong, Beijing, Beijing 100176 (CN); XIONG, LiuLin, Beijing, Beijing 100034 (CN)
(74) Representative: Thoma, Michael

(56) References cited:
- WO-A1-2016/168184
- WO-A1-2017/100412
- WO-A2-2007/143439
- CN-U- 203 776 996
- US-A1- 2016 121 143
- US-B1- 9 833 256

## Description

### Technical Field

The present invention relates to the technical field of medical devices, in particular to an ultrasonic surgical integrated knife.

### Background

With the popularity of minimally invasive surgeries, an ultrasonic scalpel has become a conventional surgical instrument. An ultrasonic scalpel uses ultrasonic energy to treat soft tissues, in which cutting and coagulation are accomplished at the same time, and minimal lateral thermal damage to tissues can be ensured. At present, an ultrasonic knife on the market consists of such separate components as a handle, a blade and an energy transducer, and thus the handle, the blade and the energy transducer need to be assembled before surgery. Since the structure is complex and the assembling process is tedious, a lot of inconvenience has been brought to doctors. An ultrasonic surgical integrated knife according to the preamble of claim 1 is known from, for example, WO 2017/100412 A1. Similar ultrasonic scalpels are known from WO 2016/168184 A1, CN 203 776 996 U and US 2016/121143 A1.

### Summary

Therefore, a technical problem to be solved in the present invention is to overcome shortcomings in the prior art that assembly of an ultrasonic scalpel before surgery is tedious and the use is inconvenient, and thus provide an ultrasonic surgical integrated knife which can be used conveniently with no need of assembly.

To solve the problem, the present invention provides an ultrasonic surgical integrated knife, including:
a handle, internally provided with an energy transducer; and
a blade assembly, including a waveguide and an outer sheath which are sleeved with each other, wherein the waveguide and the outer sheath are detachably connected, and the waveguide is fixedly connected with the energy transducer.

The ultrasonic surgical integrated knife further includes a connecting piece arranged inside the handle and configured to simultaneously connect the energy transducer and the waveguide.

The connecting piece is a fixed block fastened in the handle, the energy transducer and one end of the waveguide are installed on two opposite end faces of the fixed block through a fastening piece, and the other end of the waveguide extends out of the handle.

The outer sheath is provided with a working end and a connecting end, and the connecting end is connected with one end, close to the energy transducer, of the waveguide in a position limiting manner.

A conductive ring is arranged on one end, close to the blade assembly, of the energy transducer, and the conductive ring is provided with an oblique plane which extends along an axial direction of the energy transducer.

A distance between the oblique plane and an axis of the energy transducer gradually increases from one end close to the blade assembly to the other end far away from the blade assembly.

The handle is further provided with a conductive strip which is abutted against the conductive ring, and the conductive strip is provided with a first connecting line which is electrically connected with an external device.

The ultrasonic surgical integrated knife further includes an installation piece which is arranged at an end of the handle opposite to another end connected with the blade assembly, wherein the first connecting line is electrically connected with the external device through the installation piece.

The installation piece includes a socket and a plug which are respectively installed inside and outside the handle, and a second connecting line which is electrically connected with the external device is arranged on the plug, and is electrically connected with the first connecting line through the socket.

The technical solution of the present invention has the following advantages:
1. As to the ultrasonic surgical integrated knife provided in the present invention, an energy transducer is internally arranged in a handle, and a waveguide is fixedly connected with the energy transducer; the handle of the integrated knife produced in this way can be used for multiple times after cleaning and disinfection, while a disposable outer sheath in a blade assembly is detachably connected with the waveguide, and therefore, cross infection during surgery can be avoided after replacement of the disposable outer sheath; furthermore, the integrated knife does not need to be assembled before surgery, and such quickness for use brings great convenience to the surgery, thereby avoiding errors caused by tedious components during assembly.
2. As to the ultrasonic surgical integrated knife provided in the present invention, a connecting piece which is configured to connect the energy transducer with the waveguide is further arranged inside the handle, and the connecting piece is a fixed block which is fixed inside the handle. With such configuration, the energy transducer and the waveguide can be fixed inside the handle, and no displacement occurs due to other factors, thereby ensuring firmness of installation.
3. As to the ultrasonic surgical integrated knife provided in the present invention, the connecting end of the outer sheath is connected with one end, close to the energy transducer, of the waveguide in a position limiting manner. In this connecting manner, the outer sheath can be effectively fastened, thereby avoiding deviation of the connecting end of the outer sheath when the working end of the outer sheath moves, and ensuring stability of the outer sheath during action.
4. As to the ultrasonic surgical integrated knife provided in the present invention, a conductive ring is arranged on one end, close to the blade assembly, of the energy transducer, the conductive ring is provided with an oblique plane which extends along an axial direction of the energy transducer, and a distance between the oblique plane and an axis of the energy transducer gradually increases from one end close to the blade assembly to the other end far away from the blade assembly. Through such a design, not only can the energy transducer be more easily assembled into a fixed block during assembly, but also during assembly, the energy transducer can generate an extrusion force on the conductive ring when the energy transducer just contacts the conductive strip; moreover, the extrusion force reaches its maximum value when assembly is finished, such that the conductive strip is in good contact with the conductive ring, and poor contact is avoided.
5. As to the ultrasonic surgical integrated knife provided in the present invention, the conductive strip is further provided with a first connecting line which is electrically connected with an external device; through such a design, the energy transducer and the connecting line are disassembled into two separate components, rotation of the energy transducer will not cause rotation of the connecting line, and the blade can arbitrarily rotate to a cutting angle, thereby avoiding problems that the connecting line is liable to be entangled due to direct connection between the energy transducer and the connecting line previously and the rotation angle of the blade is limited.
6. As to the ultrasonic surgical integrated knife provided in the present invention, an installation piece is further arranged at an end of the handle opposite to another end connected with the blade assembly. The installation piece is set to be a socket inside, and set to be a plug outside. In addition to effectively connecting an internal device to a power supply, this allows omission of a power line for convenient use of the integrated knife, and a second connecting line can be used repeatedly under a premise of guaranteed quality.

### Brief Description of Drawings

In order to more clearly describe technical solutions in specific embodiments of the present invention or in the prior art, a brief introduction will be given below on the accompanying drawings which need to be used in the description of specific embodiments or the prior art. Apparently, the accompanying drawings described below merely represent some embodiments of the present invention, and for those skilled in the art, other drawings can be obtained based on these drawings without any creative effort.
Figure 1 is a schematic diagram of an ultrasonic surgical integrated knife provided in an embodiment of the present invention;
Figure 2 is a schematic diagram of an ultrasonic surgical integrated knife shown in Figure 1 with an energy transducer being removed;
Figure 3 is a schematic diagram of an ultrasonic surgical integrated knife shown in Figure 1 with a waveguide and an outer sheath being disassembled;
Figure 4 is a schematic diagram of an energy transducer of an ultrasonic surgical integrated knife shown in Figure 1;
Figure 5 is a schematic diagram of an energy transducer shown in Figure 4 after installation;

### Reference numerals:

1-blade assembly; 2-connecting piece; 3-energy transducer;
4-installation piece; 5-function key; 6-outer sheath control handle;
7-handle; 8-conductive strip; 9-conductive ring;
11-waveguide; 12-outer sheath; 13-working end;
14-connecting end; 15-first connecting line; 16-second connecting line;
17-socket; 18-plug.

### Detailed Description of the Embodiments

A clear and complete description will be given below on the technical solutions of the present invention in combination with the accompanying drawings. Apparently, the described embodiments are merely a part, but not all, of the embodiments of the present invention. Based on the embodiments in the present invention. The invention is defined by independent claim 1. Further embodiments are defined in the dependent claims.

In addition, the technical features involved in different embodiments of the present invention described below can be combined with each other as long as they do not conflict with one another.

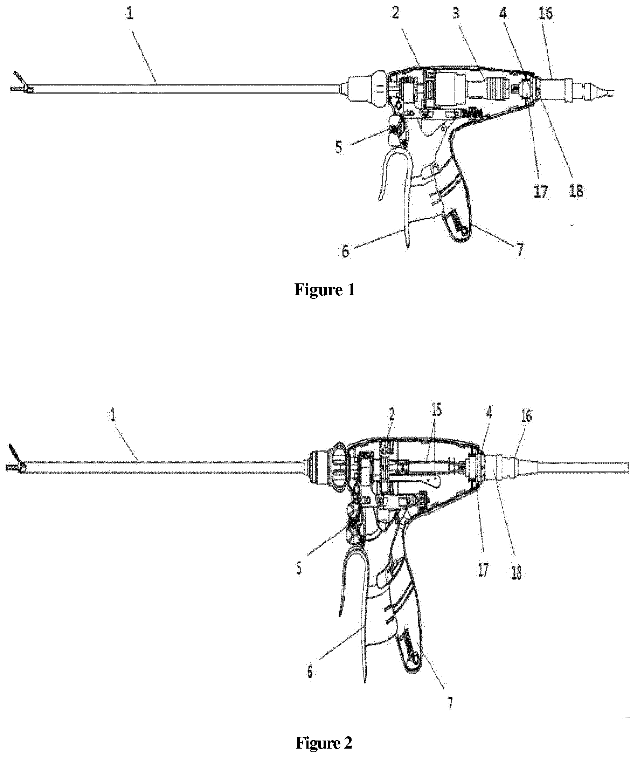

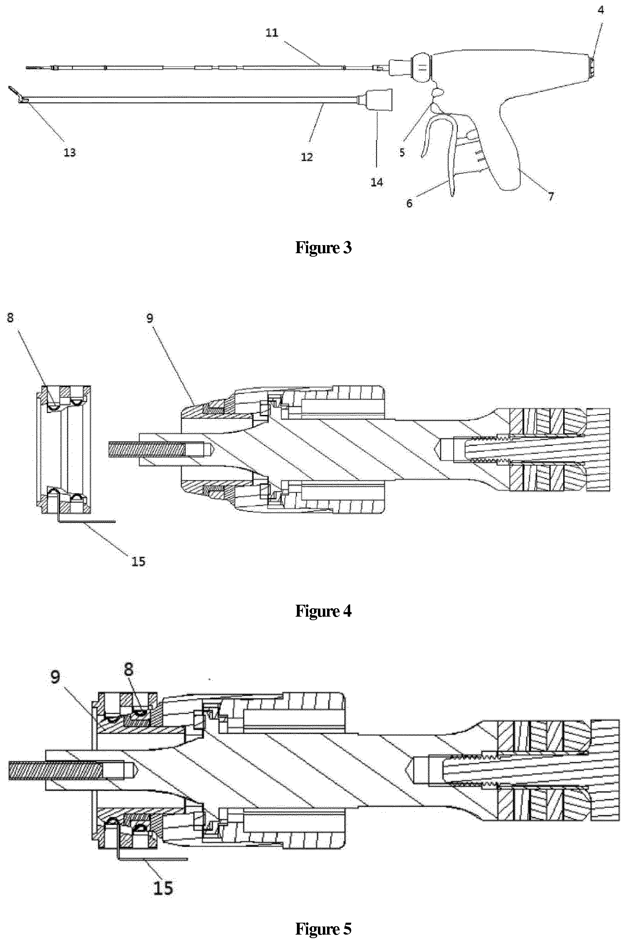

A specific embodiment of an ultrasonic surgical integrated knife shown in Figs. 1 to 5 includes a handle 7, internally provided with an energy transducer 3; and a blade assembly 1, including a waveguide 11 and an outer sheath 12 which are sleeved with each other, wherein the waveguide 11 and the outer sheath 12 are detachably connected, and the waveguide 11 is fixedly connected with the energy transducer 3.

In the present embodiment, an energy transducer 3 is internally arranged in the handle 7, and the waveguide 11 is fixedly connected with the energy transducer 3; the handle 7 of the integrated knife produced in this way can be used for multiple times after cleaning and disinfection, while the disposable outer sheath 12 in the blade assembly 1 is detachably connected with the waveguide 11, and therefore, the risk of cross infection during surgery can be avoided after replacement of the disposable outer sheath; furthermore, the integrated knife does not need to be assembled before surgery, and such quickness for use brings great convenience to the surgery, thereby avoiding errors caused by tedious components during assembly.

Specifically, in the present embodiment, as shown in Figs. 1 and 2, the handle 7 includes a main body part configured to install the energy transducer 3 and an outer sheath control handle 6 for convenient handheld operation, function keys 5 are further arranged on an outer surface of the main body part of the handle 7, doctors enter different working modes through selection of the function keys 5, and control working states of the outer sheath 12 through the outer sheath control handle 6. The outer sheath 12 is provided with a hollow cavity, a waveguide 11 is accommodated in the cavity, and is fixedly connected with the energy transducer 3, and the connecting mode can be welding, threaded connection, or the like.

In the present embodiment, the ultrasonic surgical integrated knife further includes a connecting piece 2 arranged inside the handle 7 and configured to simultaneously connect the energy transducer 3 and the waveguide 11. Specifically, the connecting piece 2 is a fixed block fastened in the handle, the fixed block is embedded at a position, close to the blade assembly 1, inside the handle 7, the energy transducer 3 and one end of the waveguide 11 are installed on two opposite end faces of the fixed block through a fastening piece, that is, axes of the energy transducer 3, the waveguide 11 and the fixed block are collinear, and the fastening piece can be a screw, and the installation and fixation can also be achieved in a form of interference fit; and the other end of the waveguide 11 is arranged to extend out of the handle 7, thereby facilitating installation and disassembly of the outer sheath 12. The fixed block can be set to fix the energy transducer 3 and the waveguide 11 inside with no displacement caused by other factors.

In the present embodiment, the outer sheath 12 is provided with a working end 13 and a connecting end 14, and a bar body configured to connect the working end 13 with the connecting end 14, and the connecting end 14 is connected with one end, close to the energy transducer 3, of the waveguide 11 in a position limiting manner, and specifically can be in groove connection. The working end 13 is a plier which can be opened and closed under the action of the outer sheath control handle 6. The connecting end of the outer sheath 12 is connected with one end, close to the energy transducer 3, of the waveguide 11 in a position limiting manner. In this connecting manner, the outer sheath 12 can be effectively fastened, thereby avoiding deviation of the connecting end 14 of the outer sheath 12 when the working end 13 of the outer sheath moves, and ensuring stability of the outer sheath 12 during action.

As an alternative embodiment, in addition to a groove connection mode, the outer sheath 12 and the waveguide 11 can also be in sleeved or spliced connection.

In the present embodiment, a conductive ring 9 is arranged on one end, close to the blade assembly 1, of the energy transducer 3, the conductive ring 9 is provided with an oblique plane which extends along an axial direction of the energy transducer 3. Specifically, a distance between the oblique plane and an axis of the energy transducer 3 gradually increases from one end close to the blade assembly 1 to the other end far away from the blade assembly 1. A conductive strip 8 which is in elastic electric connection with the conductive ring 9 is arranged at a corresponding position inside a handle 7. Through such a design, not only can the energy transducer 3 be more easily assembled into a fixed block during assembly, but also during assembly, the energy transducer 3 can generate an extrusion force on the conductive ring 9 when the energy transducer 3 just contacts the conductive strip 8; moreover, the extrusion force reaches its maximum value when assembly is finished, such that the conductive strip 8 is in good contact with the conductive ring 9, and poor contact is avoided.

Of course, a contact surface between the conductive ring 9 and the conductive strip 8 can also be a plane which is in parallel with an axial direction of the energy transducer 3.

In the present embodiment, the handle 7 is further provided with a conductive strip 8 which is abutted against the conductive ring 9, and the conductive strip 8 is an elastic strip, thereby facilitating assembly of the energy transducer 3 and the handle 7. Moreover, after they are assembled in place, an effect contact connection is maintained under an elastic action. The conductive strip 8 is provided with a first connecting line 15 which is electrically connected with an external device, and the first connecting line 15 is set to be close to an inner wall of the handle 7, and extends in a direction which is an axial direction of the handle 7. Through such a design, the energy transducer 3 and the connecting line are disassembled into two separate components, rotation of the energy transducer 3 will not cause rotation of the connecting line, and the blade can arbitrarily rotate to a cutting angle, thereby avoiding problems that the connecting line is liable to be entangled due to direct connection between the energy transducer 3 and the connecting line previously and the rotation angle of the blade is limited.

In the present embodiment, the ultrasonic surgical integrated knife further includes an installation piece 4 which is arranged at an end of the handle 7 opposite to another end connected with the blade assembly 1, and the first connecting line 15 is electrically connected with the external device through the installation piece 4. Specifically, the installation piece 4 includes a socket 17 and a plug 18 which are respectively installed inside and outside the handle 7, and the socket 17 and the plug 18 are adaptive, thereby ensuring reliability of electrical connection. A second connecting line 16 which is electrically connected with the external device is arranged on the plug 18, and is electrically connected with the first connecting line 15 through the socket 17. In addition to effectively connecting an internal device to a power supply, this allows omission of a power line for convenient use of the integrated knife, and the second connecting line 16 can be used repeatedly under a premise of guaranteed quality.

As an alternative embodiment, the first connecting line 15 can also extend out of the handle 7 directly through an opening at an end of a handle 7, so as to be in electrical connection with an external device, for example, a motor, etc.

In addition, as shown in Figure 1, the positions or forms of the socket 17 and the plug 18 can be interchanged; preferably, to facilitate overall convenience of the integrated knife, a form that the socket 17 is arranged inside and the plug 18 is arranged outside is adopted.

During specific use, since before delivery, the waveguide 11, the energy transducer 3 and the handle 7 are integrated into one piece, and therefore, an assembly step before surgery is omitted, and the integrated knife can be used just after the outer sheath 12 is sleeved along an axial direction of the waveguide 11 and is fastened at the connecting end 14. Moreover, in the using process, since the first connecting line 15 is not in direct connection with the energy transducer 3, but is fixed on the conductive strip 8 in the handle 7, therefore, even if a blade assembly 1 drives the energy transducer 3 to rotate during surgery, since the handle 7 will not rotate, the first connecting line 15 installed on the handle will be fixed at its current position and will not be entangled, thereby ensuring normal operation of the surgery.

Obviously, the above embodiments are merely examples for clear description, rather than for limiting implementations. For those skilled in the art, other changes or variations in different forms can also be made on the basis of the above description. There is no need or possibility of enumerating all implementations in an exhaustive manner.

## Claims

1. An ultrasonic surgical integrated knife, comprising:
a handle (7), internally provided with an energy transducer (3); and
a blade assembly (1), comprising a waveguide (11) and an outer sheath (12) which are sleeved with each other, wherein the waveguide (11) and the outer sheath (12) are detachably connected, and the waveguide (11) is fixedly connected with the energy transducer (3),
wherein a conductive ring (9) is arranged on one end, close to the blade assembly (1), of the energy transducer (3),
**characterized in that**
the waveguide (11) is fixedly connected with the energy transducer (3) by welding connection, and the conductive ring (9) is provided with an oblique plane which extends along an axial direction of the energy transducer (3), wherein a distance between the oblique plane and an axis of the energy transducer (3) gradually increases from one end close to the blade assembly (1) to the other end far away from the blade assembly (1),
and the handle (7) is further provided with a conductive strip (8) which is abutted against the conductive ring (9).

2. The ultrasonic surgical integrated knife of claim 1, wherein the outer sheath (12) is provided with a working end (13) and a connecting end (14), and the connecting end (14) is connected with one end, close to the energy transducer (3), of the waveguide (11) in a position limiting manner.

3. The ultrasonic surgical integrated knife of claim 1, wherein the conductive strip (8) is provided with a first connecting line (15) which is electrically connected with an external device.

4. The ultrasonic surgical integrated knife of claim 3, further comprising an installation piece (4) which is arranged at an end of the handle (7) opposite to another end connected with the blade assembly (1), wherein the first connecting line (15) is electrically connected with the external device through the installation piece (4).

5. The ultrasonic surgical integrated knife of claim 4, wherein the installation piece (4) comprises a socket (17) and a plug (18) which are respectively installed inside and outside the handle (7), and a second connecting line (16) which is electrically connected with the external device is arranged on the plug (18), and is electrically connected with the first connecting line (15) through the socket (17).

## Patentansprüche

1. Integriertes chirurgisches Ultraschallmesser, umfassend:
einen Griff (7), der innen mit einem Energiewandler (3) versehen ist; und
eine Klingenbaugruppe (1), die einen Wellenleiter (1) und eine äußere Ummantelung (12) umfasst, die sich umhüllen, wobei der Wellenleiter (11) und die äußere Ummantelung (12) lösbar verbunden sind und der Wellenleiter (11) fest mit dem Energiewandler (3) verbunden ist, wobei ein leitfähiger Ring (9) an einem nahe der Klingenanordnung (1) gelegenen Ende des Energiewandlers (3) angeordnet ist,
**dadurch gekennzeichnet, dass** der Wellenleiter (11) durch eine Schweißverbindung fest mit dem Energiewandler (3) verbunden ist und der leitfähige Ring (9) mit einer schiefen Ebene versehen ist, die sich entlang einer axialen Richtung des Energiewandlers (3) erstreckt, wobei ein Abstand zwischen der schiefen Ebene und einer Achse des Energiewandlers (3) von einem nahe der Klingenbaugruppe (1) gelegenen Ende zu dem anderen, von der Klingenbaugruppe (1) entfernten Ende allmählich größer wird, und dass der Griff (7) ferner mit einem leitfähigen Streifen (8) versehen ist, der gegen den leitfähigen Ring (9) stößt.

2. Integriertes chirurgisches Ultraschallmesser nach Anspruch 1, wobei die äußere Ummantelung (12) mit einem Arbeitsende (13) und einem Verbindungsende (14) versehen ist und das Verbindungsende (14) mit einem nahe dem Energiewandler (3) gelegenen Ende des Wellenleiters (11) in einer lagebegrenzenden Weise verbunden ist.

3. Integriertes chirurgisches Ultraschallmesser nach Anspruch 3, wobei der leitfähige Streifen (8) mit einer ersten Verbindungsleitung (15) versehen ist, die mit einem externen Gerät elektrisch verbunden ist.

4. Integriertes chirurgisches Ultraschallmesser nach Anspruch 3, ferner mit einem Einbaustück (4), das an einem Ende des Handgriffs (7) angeordnet ist, das einem anderen mit der Klingenbaugruppe (1) verbundenen Ende entgegengesetzt ist, wobei die erste Verbindungsleitung (15) durch das Einbaustück (4) mit dem externen Gerät elektrisch verbunden ist.

5. Integriertes chirurgisches Ultraschallmesser nach Anspruch 4, wobei das Einbaustück (4) eine Buchse (17) und einen Stecker (18) umfasst, die jeweils innerhalb und außerhalb des Handgriffs (7) montiert sind, und eine zweite Verbindungsleitung (16), die mit dem an dem Stecker (18) angeordneten externen Gerät elektrisch verbunden ist und durch die Buchse (17) mit der ersten Verbindungsleitung (15) elektrisch verbunden ist.

## Revendications

1. Couteau chirurgical intégré à ultrasons, comprenant :
une poignée (7), pourvue intérieurement d'un convertisseur d'énergie (3) ; et
un ensemble de lame (1), comprenant un guide d'ondes (11) et une gaine extérieure (12), qui sont emmanchés l'un dans l'autre, dans lequel le guide d'ondes (11) et la gaine extérieure (12) sont reliés de manière amovible, et le guide d'ondes (11) est relié de manière fixe au convertisseur d'énergie (3), un anneau conducteur (9) étant agencé sur une extrémité, près de l'ensemble de lame (1), du convertisseur d'énergie (3),
**caractérisé en ce que** le guide d'ondes (11) est relié de manière fixe au convertisseur d'énergie (3) par assemblage soudé, et l'anneau conducteur (9) est pourvu d'un plan oblique qui s'étend le long d'une direction axiale du convertisseur d'énergie (3), une distance entre le plan oblique et un axe du convertisseur d'énergie (3) augmentant progressivement d'une extrémité près de l'ensemble de lame (1) à l'autre extrémité éloignée de l'ensemble de lame (1),
et la poignée (7) est en outre pourvue d'une bande conductrice (8) qui est en butée contre l'anneau conducteur (9).

2. Couteau chirurgical intégré à ultrasons selon la revendication 1, dans lequel la gaine extérieure (12) est pourvue d'une extrémité de travail (13) et une extrémité de connexion (14), et l'extrémité de connexion (14) est reliée à une extrémité, près du convertisseur d'énergie (3), du guide d'ondes (11) de manière à limiter la position.

3. Couteau chirurgical intégré à ultrasons selon la revendication 1, dans lequel la bande conductrice (8) est pourvue d'une première ligne de connexion (15) qui est connectée électriquement à un dispositif externe.

4. Couteau chirurgical intégré à ultrasons selon la revendication 3, comprenant en outre une pièce d'installation (4) qui est agencée sur une extrémité de la poignée (7) opposée à une autre extrémité reliée à l'ensemble de lame (1), dans lequel la première ligne de connexion (15) est connectée électriquement au dispositif externe par la pièce d'installation (4).

5. Couteau chirurgical intégré à ultrasons selon la revendication 4, dans lequel la pièce d'installation (4) comprend une douille (17) et une fiche (18), qui sont respectivement installées à l'intérieur et à l'extérieur de la poignée (7), et une deuxième ligne de connexion (16), qui est connectée électriquement au dispositif externe, est agencée sur la fiche (18) et est connectée électriquement à la première ligne de connexion (15) par la douille (17).
